# FASCICULE DE BREVET EUROPEEN

(11) **EP 2 680 876 B1**
(45) Date de publication et mention de la délivrance du brevet: **04.11.2015**
(21) Numéro de dépôt: 12712302.4
(22) Date de dépôt: 29.02.2012
(51) Int. Cl.: A61K 38/48, C07K 14/745, A61P 7/04

(54) **FACTEUR XA DEPOURVU DE DOMAINE GLA POUR LE TRAITEMENT DES HEMOPHILIES A OU B AVEC OU SANS ANTICORPS**
GLA-DOMAINLOSER FAKTOR XA FÜR DIE BEHANDLUNG VON HÄMOPHILIE A ODER B MIT ODER OHNE ANTIKÖRPER
GLA-DOMAINLESS FACTOR XA FOR TREATING HAEMOPHILIA A OR B WITH OR WITHOUT ANTIBODY

(30) Priorité: 01.03.2011 FR 1151637
(43) Date de publication de la demande: 08.01.2014
(73) Titulaire: Universite Joseph Fourier (Grenoble 1), 38041 Grenoble (FR); Centre National de la Recherche Scientifique, 75794 Paris Cedex 16 (FR)
(72) Inventeur: POLACK, Benoît, F-38950 Saint Martin Le Vinoux (FR); THOMAS, Aline, F-38240 Meylan (FR)
(74) Mandataire: Agasse, Stéphane
(86) Numéro de dépôt international: PCT/FR2012/050425
(87) Numéro de publication internationale: WO 2012/117203

(56) Documents cités:
- WO-A2-2009/042962
- SKOGEN ET AL: "Comparison of coagulation factor Xa and des-(1-44)factor Xa in the assembly of prothrombinase.", JOURNAL OF BIOLOGICAL CHEMISTRY, vol. 259, no. 4, 1 février 1984 (1984-02-01), pages 2306-2310, XP055006590, ISSN: 0021-9258
- ZERA TELLIER ET AL: "Management of Haemophilia A-Inhibitor Patients: Clinical and Regulatory Perspectives", CLINICAL REVIEWS IN ALLERGY & IMMUNOLOGY, vol. 37, no. 2, 28 janvier 2009 (2009-01-28), pages 125-134, XP055006650, ISSN: 1080-0549, DOI: 10.1007/s12016-009-8115-4
- VENKATESWARLU D ET AL: "STRUCTURE AND DYNAMICS OF ZYMOGEN HUMAN BLOOD COAGULATION FACTOR X", BIOPHYSICAL JOURNAL, BIOPHYSICAL SOCIETY, US, vol. 82, no. 3, 1 mars 2002 (2002-03-01), pages 1190-1206, XP009069303, ISSN: 0006-3495
- R Marlu ET AL: "1ère Journée Scientifique du Médicament : Nouvelles Stratégies Thérapeutiques", , 23 juin 2011 (2011-06-23), pages FP-1-43, XP55006663, Grenoble, France Extrait de l'Internet: URL:http://dpm.ujf-grenoble.fr/jsm/documen ts/PDFresumes_poster2011.pdf [extrait le 2011-09-09]

## Description

La présente invention a pour objet une composition pharmaceutique comprenant un facteur Xa modifié (GDXa) - ledit GDXa est un FXa dépourvu de domaine Gla non thrombogène et qui peut se lier à l'inhibiteur de la voie du facteur tissulaire (TFPI)) mais ne possède pas de site de liaison aux phospholipides - pour l'utilisation dans la prévention ou le traitement d'un accident hémorragique chez un patient atteint d'hémophilie A ou B avec ou sans anticorps dirigés contre le facteur VIII ou le facteur IX.

L'hémophilie A comme l'hémophilie B regroupe deux types d'hémophilie, l'hémophilie constitutionnelle et l'hémophile acquise.

L'hémophilie constitutionnelle de type A est une maladie hémorragique caractérisée par un déficit quantitatif ou qualitatif en FVIII résultant d'une anomalie du gène du FVIII. L'hémophilie constitutionnelle de type B est également une maladie hémorragique mais caractérisée par un déficit quantitatif ou qualitatif en FIX résultant d'une anomalie du gène du FIX.

L'hémophilie acquise de type A ou B se définit par l'apparition d'auto anticorps dirigés contre ces FVIII ou FIX.

L'hémophilie se traduit par un déficit de la coagulation sanguine en réponse à une hémorragie. Les hémophiles de type A ou B non traités présentent des symptômes tels que des saignements excessifs en cas de blessure et parfois même des hémorragies spontanées.

L'activité biologique des facteurs VIII ou IX s'évalue en pourcentage de la normale. L'individu normal étant considéré comme ayant 100% d'activité. Si l'activité est indétectable (inférieure à 1%), il s'agit d'une hémophilie sévère, si l'activité est comprise entre 1 et 5%, l'hémophilie est dite modérée; au-delà et jusqu'à 30% l'hémophilie est mineure.

Les patients atteints d'hémophilie A et B peuvent être traités par des concentrés comprenant respectivement du FVIII ou du FIX qui peuvent être des dérivés plasmatiques ou des produits issus du génie génétique. Ces concentrés peuvent être administrés à l'occasion de chaque hémorragie, dans ce cas, il convient de commencer le traitement le plus rapidement possible, à l'apparition des premiers signes. Le traitement peut également être administré de façon prophylactique, régulièrement 2 à 3 fois par semaine de façon à prévenir les hémorragies. Cependant, le traitement peut donner lieu à l'apparition d'anticorps dirigés contre le FVIII ou le FIX appelés inhibiteurs. La présence de tels anticorps rend alors inefficace les administrations de facteurs VIII ou IX. Ces anticorps sont des immunoglobulines de classe G à prédominance IgG4. Ils se développent tôt dès les premières administrations, souvent avant la dixième. Certains patients restent de faibles répondeurs (titre d'anticorps < 10 U.B), d'autres appelés forts répondeurs atteignent des titres qui ne permettent plus de les traiter avec le facteur correspondant.

A la date de la présente invention, il n'existe pas de traitement permettant de prévenir et ou traiter de façon satisfaisante l'existence d'un risque hémorragique chez les patients atteints d'hémophilie A ou B et présentant un inhibiteur. En effet, les produits disponibles peuvent être inefficaces (Astermark J, Donfield SM, DiMichele DM, Gringeri A, Gilbert SA, Waters J, Berntorp E, for the FSG. A randomized comparison of bypassing agents in hemophilia complicated by an inhibitor: the FEIBA NovoSeven Comparative (FENOC) Study. Blood. 2007; 109: 546-51) ou leur administration compliquée d'accidents thrombotiques (Aledort LM. Comparative thrombotic event incidence after infusion of recombinant factor VIIa versus factor VIII inhibitor bypass activity. J Thromb Haemost. 2004; 2: 1709.).

Il existe donc un besoin avéré pour des alternatives thérapeutiques aux traitements existant. Cependant, la mise au point d'un tel traitement s'est avéré très difficile, celui-ci devant :
- permettre d'arrêter l'hémorragie,
- ne pas provoquer de thrombose,
- permettre le traitement ou la prévention de l'accident hémorragique même en présence d'anticorps anti FVIII ou FIX.

La présente invention répond à ce besoin, elle a pour objet une composition pharmaceutique comprenant un facteur X activé (FXa) modifié ledit FXa modifié (GDXa) - étant non thrombogène et pouvant se lier à l'inhibiteur de la voie du facteur tissulaire (TFPI) mais ne possédant pas de site de liaison aux phospholipides - pour l'utilisation dans la prévention ou le traitement d'un accident hémorragique chez un patient atteint d'hémophilie A ou B avec ou sans anticorps dirigés contre le facteur VIII ou le facteur IX.

Les anticorps sont apparus soit à la suite d'un traitement par des facteurs FVIII ou FIX ou spontanément comme dans l'hémophilie acquise.

Dans le cadre de la présente invention, la mention du facteur FXa fait référence au facteur X activé obtenu par activation du Facteur X natif naturellement présent dans le plasma ou à l'état isolé dans sa forme originelle, non modifiée. Ce terme englobe les FX isolés à partir du plasma mais également les FX produits de façon recombinante ou obtenus par synthèse chimique qui ont été activés. Le facteur Xa ou facteur Xa (FXa) natif fait référence, dans le cadre de la présente invention à une protéine de type sérine protéase intervenant dans la coagulation et qui est produite sous une forme inactive le facteur X (FX).

L'activation du facteur X de la coagulation est une étape clef dans la coagulation du sang et l'arrêt des hémorragies. Son activation est nécessaire pour les étapes de propagation et d'amplification de la coagulation. Son activation est également nécessaire pour l'arrêt de l'activation de la coagulation par l'intermédiaire de son interaction avec le TFPI.

Le FX est activé soit par le facteur IX activé et son cofacteur, le facteur VIII activé ou par le facteur VII activé et son cofacteur, le facteur tissulaire (FT). Le FXa forme le complexe prothrombinase, lequel est lié aux membranes avec le facteur V activé et est le composant actif dans le complexe prothrombinase qui catalyse la conversion de la prothrombine en thrombine. La thrombine catalyse quant à elle, la conversion du fibrinogène en fibrine qui conduit à la formation des caillots dans le sang et à l'arrêt des saignements. L'activité du FXa peut être appelée « activité procoagulante ».

Leytus et al., (Biochemistry, 1986, 25 :5098-5102) ainsi que Venkateswarlu et al., (Biophysical Journal, 2002, 82 :1190-1206) décrivent le facteur X et les différents domaines présents dans ce polypeptide. Un clivage catalytique de la chaine lourde permet l'activation du FX en FXa. Le FXa comporte une chaine légère dont un exemple est représenté par l'identifiant de séquence SEQ ID No. 1 et une chaine lourde dont un exemple est représenté par l'identifiant de séquence SEQ ID No. 2.

Dans le cadre de la présente invention, le terme FXa modifié désigne des FXa ne pouvant plus se lier aux phospholipides, ne possédant plus d'activité procoagulante ou une activité procoagulante réduite. Dans le cadre de la présente invention, un tel facteur est nommé GDXa. L' « activité procoagulante » est définie comme la capacité d'un facteur à causer la coagulation sanguine ou la formation de caillots. Une activité procoagulante réduite signifie que l'activité est réduite d'au moins 50%, de préférence au moins 90% et de façon encore plus préférée de plus de 95% par rapport à l'activité du FXa natif.

Le facteur Xa modifié selon l'invention, GDXa, est dépourvu de son domaine γ-carboxyglumatic acide (Gla) de liaison aux phospholipides. Les 43 premiers acides aminés de la chaine légère (résidus 1-43 de SEQ ID No. 1) représentent le domaine Gla car il contient 11 résidus modifiés de façon post traductionnelle (acide γ-carboxyglutamique). Une digestion par la chymotrypsine permet de supprimer les résidus 1-43 permettant de générer un FXa dépourvu de son domaine de fixation aux phospholipides ou GDXa (pour Gla Domainless FXa). Un tel GDXa peut également comprendre d'autres modifications en plus de l'absence de son domaine Gla. Un tel FXa modifié conserve des propriétés de liaison au facteur Va mais ne possède pas d'activité procoagulante. Un exemple de GDXa est représenté par l'identifiant de séquence SEQ ID No. 7 ou encore SEQ ID No. 3 pour sa chaine légère et par SEQ ID No. 2 pour sa chaine lourde. Cette absence d'activité procoagulante peut être définie par son incapacité à activer la coagulation quand il est ajouté à du plasma en absence de facteur tissulaire, ce qui le différencie du facteur Xa natif.

Le GDXa peut être obtenu par clivage du facteur X par protéolyse ménagée par la chymotrypsine et activation par une protéase spécifique selon l'une des méthodes usuelles telle celle décrite par Skogen *et al*., (1984). Un exemple de GDX avant son activation peut être représenté par l'identifiant de séquence SEQ ID No. 20 pour sa séquence nucléotidique et SEQ ID No. 28 pour sa séquence en acides aminés.

Le GDXa peut être produit par synthèse chimique, soit sous la forme d'une seule séquence, soit sous la forme de plusieurs séquences qui sont ensuite liées les unes aux autres. Cette synthèse peut se faire en phase solide ou en solution. Ces techniques sont plus particulièrement décrites par Atherton and Shepard in "Solid phase peptide synthesis" (IRL press Oxford, 1989) et par Houbenweyl (in "Methoden der organischen Chemie" [Methods in Organic Chemistry] published by E.Wunsch Vol. 15-1 and 11, Stuttgart, 1974), ainsi que dans les articles suivant: P. E. Dawson et al. (Science 1994; 266(5186), pp776-779); G G Kochendoerfer *et al*. (1999; 3(6), pp 665-671); P E Dawson et al. (2000, 69, Annu. Rev. Biochem., pp 923-960).

Le GDXa est non thrombogène, son absence d'activité procoagulante peut être définie par un test mesurant la génération de thrombine (Hemker et al., Thrombosis and Haemostasis. 1993 ; 70 : 617-624, cf. exemple 4).

La capacité de liaison au TFPI du GDXa est définie à l'aide de tout test bien connu de l'homme du métier. Un tel test est décrit à l'exemple 1, il met en oeuvre un substrat chromogénique permettant de déterminer le pourcentage d'inhibition du FXa et GDXa par le TFPI.

Dans un autre aspect de l'invention le GDXa est dépourvu de son domaine Gla mais également du domaine EGF1 (Epidermal Growth Factor 1). Une telle mise en oeuvre de l'invention peut être représentée par une composition comprenant un GDXa représenté par SEQ ID No 4 pour sa chaine légère et par SEQ ID No. 2 pour sa chaine lourde.

Dans un autre aspect de l'invention le GDXa est dépourvu de son domaine Gla mais également du domaine EGF2 (Epidermal Growth Factor 2), un exemple d'un tel GDXa est représenté par l'identifiant de séquence SEQ ID No 5 pour sa chaine légère et par SEQ ID No. 2 pour sa chaine lourde.

Dans un autre aspect de l'invention le GDXa est dépourvu de son domaine Gla mais également des domaines EGF1 et EGF2, un exemple d'un tel GDXa est représenté par l'identifiant de séquence SEQ ID No 6 pour sa chaine légère et par SEQ ID No. 2 pour sa chaine lourde.

Selon un autre aspect de l'invention, le GDXa est constitué uniquement par la chaine lourde du FXa. Selon un mode particulier de réalisation de l'invention, un tel FXa est représenté par l'identifiant de séquence SEQ ID No 2.

Dans un aspect encore différent, le GDXa est constitué de variants moléculaires présentant des mutations. Ainsi, différentes mutations ont été introduites dans le gène codant pour le GDXa permettant de garder une activité de génération de thrombine et réduisant l'activité enzymatique sur petits substrats peptidiques des mutants. Ces mutations peuvent être introduites par utilisation du kit QuickChange (Stratagene) et en suivant les recommandations du fabricant et selon la publication Wang & Malcolm (1999) - BioTechniques, 26 : 680-682. Ces mutations peuvent porter sur l'arginine 142 de la chaine lourde du GDXa (numérotation selon SEQ ID No. 2) qui peut être mutée pour donner tout autre acide aminé, préférentiellement la phénylalanine (par exemple SEQ ID No 10), la glycine (par exemple SEQ ID No 11), l'isoleucine (par exemple SEQ ID No 12) ou la tyrosine (par exemple SEQ ID No 13). Cette mutation peut porter sur le remplacement de la séquence peptidique du FXa humain Arg-Gln-Ser-Thr-Arg-Leu (139-143 de la chaine lourde) par la séquence équivalente provenant du FXa bovin : Arg-Leu-Ser-Ser-Thr-Leu (par exemple SEQ ID No 26). Similairement la lysine 82 de la chaine lourde (numérotation selon SEQ ID No. 2) peut également être remplacée par un acide aminé tel que la tyrosine (par exemple SEQ ID No 9).

La séquence nucléotidique codant pour le GDXa peut être synthétisée par voie chimique (Young L and Dong Q., 2004,-Nucleic Acids Res., Apr 15;32(7), Hoover,D.M. and Lubkowski,J. 2002,. Nucleic Acids Res., 30, Villalobos A, et al., 2006. BMC Bioinformatics, Jun 6;7:285). La séquence nucléotidique codant pour le GDXa peut être également amplifiée par PCR en utilisant des amorces adaptées.

Le GDXA peut également être produit par des techniques de génie génétique bien connues de l'homme du métier. La séquence nucléotidique codant pour le facteur X humain peut ainsi être clonée dans un vecteur d'expression ; la partie de la séquence nucléotidique codant pour le peptide signal, le propeptide et domaine Gla est délétée, un peptide signal est fusionné, comme celui du TIMP-1 (Crombez *et al*., 2005). Le facteur X modifié ainsi produit peut être activé soit par le complexe TF-FVlla, soit par tout autre enzyme clivant la liaison entre l'arginine 234 et l'isoleucine 235 (numérotation selon Swiss-Prot : P00742.2). Alternativement, le GDXa peut être produit directement par insertion d'une séquence de clivage reconnue par les furines ou toute autre enzyme intracellulaire, directement en amont de l'isoleucine 235 ; une séquence codant pour ces acides aminés tels que arginine-lysine-arginine permet un clivage par les furines (Nakayama et al., 1997). Pour améliorer le clivage, une séquence arginine-lysine-arginine- arginine-lysine-arginine peut être introduite. L'ADN codant pour un tel FX modifié est inséré dans un plasmide d'expression et inséré dans une lignée cellulaire ad hoc pour sa production (par exemple la lignée HEK-393E), la protéine ainsi produite étant ensuite purifiée par chromatographie.

Ces techniques sont décrites en détail dans les manuels de référence : Molecular cloning : a laboratory manual, 3ème édition- Sambrook and Russel eds., (2001) et Current Protocols in Molecular Biology - Ausubel et al. eds (2007).

Ainsi, les GDXa peuvent être également représentés par leur séquences nucléotidiques codant pour les GDXa citées ci-dessus, ainsi de telles séquences sont représentées par les identifiants de séquence suivant : SEQ ID No. 8, SEQ ID No 16 à SEQ ID No 19 pour les chaines légères et par SEQ ID No. 15 ou SEQID No. 21 à 25 et SEQ ID No. 27 pour la chaine lourde.

Un tel facteur modifié est bien connu de l'art antérieur (Morita et Jackson, 1986 ; Skogen *et al*., 1984, Padmanabhan et al., 1993. J. Mol. Biol., 232 : 947-966 ou US2009/2298119).

La composition pharmaceutique selon la présente invention peut être formulée sous toute forme galénique nécessaire à son administration. En particulier, s'agissant d'administration par voie systémique, la composition selon l'invention peut être formulée sous forme de poudre lyophilisée stérile pour injection. Les compositions pharmaceutiques selon la présente invention peuvent également être administrées par voie nasale ou parentérale. Elles pourront donc comprendre, en plus des principes actifs, tout adjuvant de formulation pharmaceutiquement acceptable, connu de l'homme du métier et qui est nécessaire à la préparation de la composition pharmaceutique sous la forme souhaitée et notamment tout excipient capable de stabiliser la protéine GDXa lyophilisée après reconstitution par une solution aqueuse pour son injection ultérieure.

Dans le cas d'un accident hémorragique, la composition pharmaceutique selon la présente invention pourra être administrée à une concentration 10 à 20 fois plus faible que le FVIIa recombinant (Novoseven®), lequel est administré selon une posologie allant de 90 à 270 µg/kg et par dose administrée. Ainsi, selon un autre aspect, la présente invention a également pour objet une composition pharmaceutique comprenant le GDXa non thrombogène et pouvant se lier au TFPI mais ne pouvant pas se lier aux phospholipides pour une administration au patient atteint d'hémophilie A ou B avec ou sans inhibiteur de 4.5 à 27 µg/kg par dose administrée. Cette administration peut être faite par voie systémique, nasale ou parentérale.

### Légendes des figures

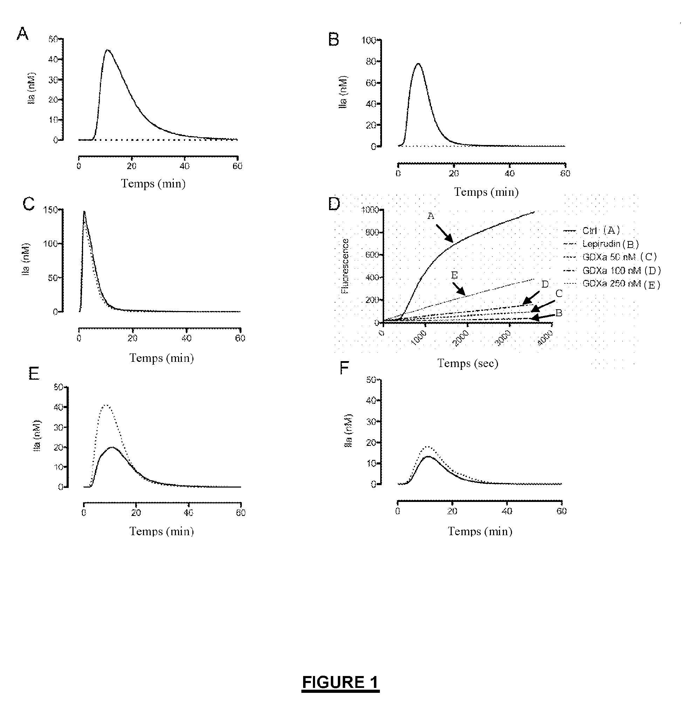

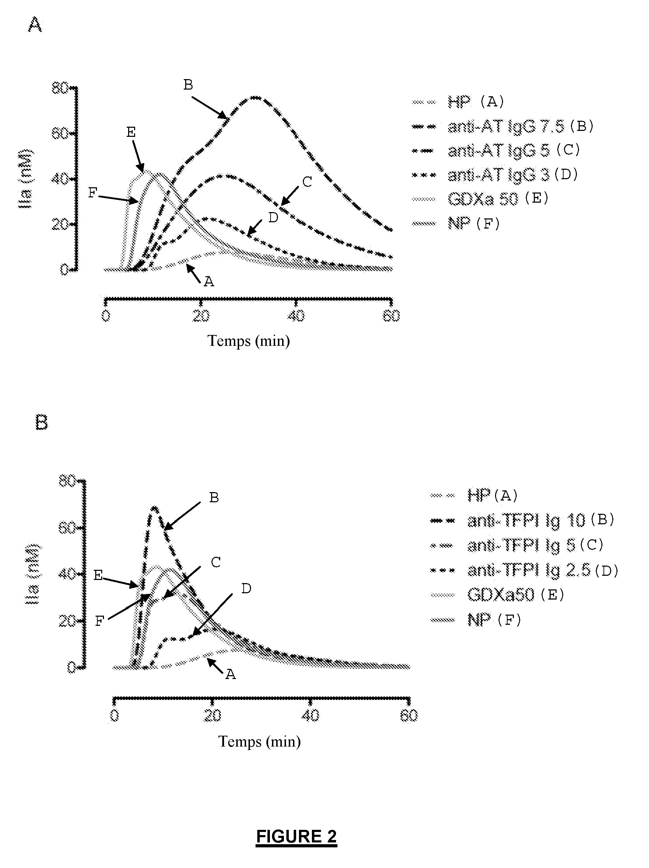

**Figure 1****.** Influence du GDXa ou FXa sur la génération de thrombine (A) Essais de production de thrombine en présence de phospholipides et de FT dans un pool de plasma normal (ligne continue) et dans un pool de plasmas d'hémophilie A sévère (ligne pointillée). (B) plasma d'hémophile enrichi avec du GDXa (50 nM) en présence de phospholipides et FT (trait plein) et sans FT (ligne pointillée). (C) plasma d'hémophile enrichi avec le FXa (50 nM) en présence de phospholipides avec FT (trait plein) et sans FT (ligne pointillée). (D) clivage du substrat ZGGR-AMC par le GDXa. Plasma normal prétraité-CTI testé en présence de phospholipides et de FT avec et sans (Ctrl- courbe A) lépirudine 6 pg/ml en l'absence (courbe B) ou en présence de diverses concentrations de GDXa (courbes C à E). (E) Génération de thrombine en présence de phospholipides et de FT dans le plasma d'hémophile enrichi avec avec 10 nM (ligne continue) ou 20 nm (ligne pointillée) de GDXa et (F) avec 40 nM (ligne épaisse) ou 200 nm (ligne pointillée) de rFVIIa.
   Les données sont représentatives des expériences réalisées avec au moins trois plasmas différents d'hémophile différents sévères de type A
**Figure 2****:** Influence des anticorps anti-AT et anti-TFPI sur la génération de thrombine dans des plasmas d'hémophiles.
   A un plasma d'hémophile A sévère a été ajouté des concentrations différentes d'anticorps anti-antithrombine humaine (A) ou anti-TFPI humain (B) puis restés pour la génération de thrombine. Les concentrations d'anticorps sont exprimées en g/l pour les anticorps anti-antithrombine et en mg/l pour les anticorps anti-TFPI. La concentration en GDXa est en nM. HP représente le plasma d'hémophile ; NP, le plasma normal.
   (A) La courbe A représente le plasma d'hémophile, les courbes B à D différentes concentrations d'anticorps anti-thrombine ajoutées au plasma d'hémophile ; la courbe E représente l'ajout de 50 nM de GDXa ajouté au plasma d'hémophile, la courbe F, représente le plasma normal.(B) La courbe A représente le plasma d'hémophile, les courbes B à D différentes concentrations d'anticorps anti-TFPI ajoutées au plasma d'hémophile ; la courbe E représente l'ajout de 50 nM de GDXa ajouté au plasma d'hémophile, la courbe F, représente le plasma normal.

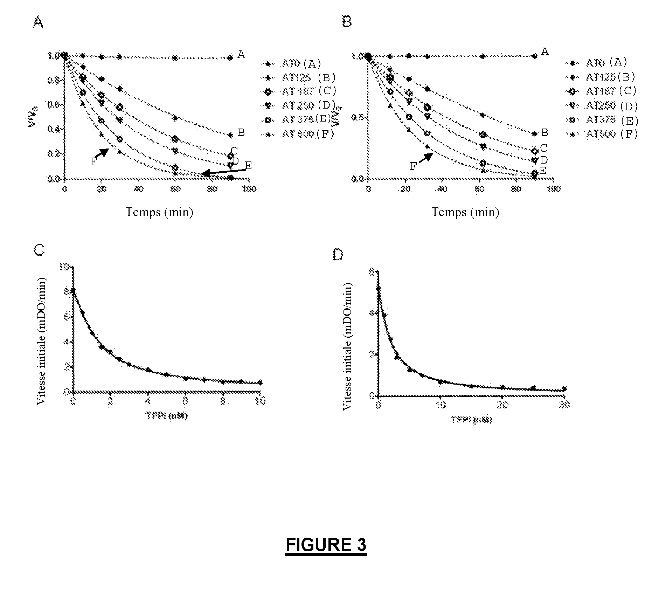

**Figure 3** **:** Inhibition enzymatique de GDXa et FXa par le TFPI ou l'antithrombine
   (A-B) pour l'inhibition d'antithrombine, 1,25 nM de FXa (A) ou GDXa (B) ont été incubés en présence de concentrations croissantes d'antithrombine (AT : 0 (courbe A) à 500 nM (courbe F)) à 37°C. Des aliquotes ont été prélevées à des moments différents (0 à 90 min) et ajoutées au substrat chromogène PNAPEP 1025.
   (C-D) L'inhibition de l'activité du FXa (C) ou GDXa (D) par le TFPI a été analysée par l'incubation d'enzyme de 0,25 nM à 25°C durant 3h dans le tampon A en présence de concentrations croissantes de TFPI (0 à 30 nM pour GDXa et 0 à 10 nM pour FXa).
   Les données sont représentatives de deux expériences différentes.

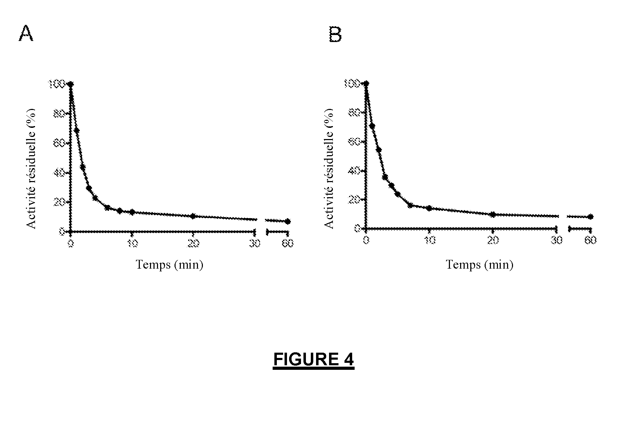

**Figure 4** **:** Détermination des propriétés pharmacocinétiques de GDXa et FXa dans le plasma
   Au plasma normal a été ajouté 50 nM FXa (A) ou GDXa (B), l'ensemble a été incubé à 37°C. Des aliquotes ont été prélevées à différents intervalles de temps et immédiatement diluées 25 fois dans le tampon A pour la détermination de l'activité résiduelle. Les demi-vies étaient de 1,4 ± 0,1 min pour FXa et 1,8 ± 0,1 min pour GDXa.
   Les données sont la moyenne de deux expériences différentes.

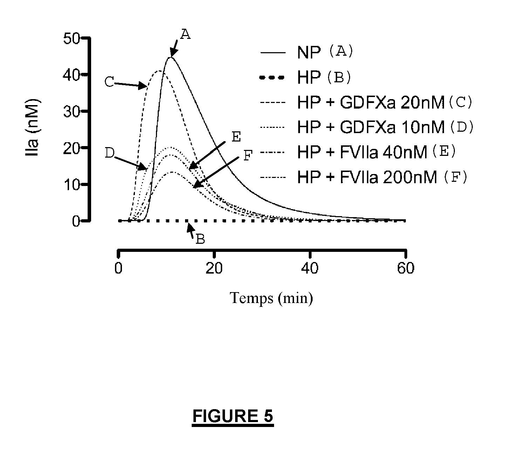

**Figure 5** **:** correction de la génération de thrombine par le rVIIa et GDXa
   Ajout de GDXa (courbes C et D) ou rVIIa à différentes concentrations (courbes E et F) dans un plasma d'hémophile (HP, courbe B). NP : Plasma normal (courbe A).

La présente invention sera illustrée par les exemples suivants.

### Exemples

### Exemple 1 : Matériel et méthodes

### Matériel :

Les pools de plasma congelé provenant de patients normaux et des plasmas individuels de patients atteints d'hémophilie A ou hémophilie B, les phospholipides TGT, le Prionex, le Corn Trypsin Inhibitor (CTI), le substrat chromogène PNAPEP 1025, le facteur Xa humain, le GDXa humain, les anticorps de mouton anti-thrombine humaine ont été obtenus par Cryopep (Montpellier, France). Le TFPI recombinant humain a été obtenu à partir Sino Biolocal Inc. (Beijing, Chine). Le facteur tissulaire recombinant humain relipidé (TF, Innovin) provient de Siemens Healthcare Diagnostics (Puteaux, France). Pour les essais de génération de thrombine, le calibrateur thrombine, FluCaKit et des plaques de microtitration de 96 puits à fond rond (Immulon 2HB, plaque de fond en U) de Diagnostica Stago (Asnières, France) ont été utilisés. Pour les expériences enzymatiques, des plaques de microtitration de 96 puits à fond plat étaient de Greiner (Frickenhausen, Allemagne) anticorps de mouton anti-TFPI proviennent de Affinity Biologicals (Sandhill Drive, Canada). Pour la détermination de l'activité TFPI, le test Actichrom activité TFPI d'American Diagnostica (Stamford, Etats-Unis)a été utilisé. Dosage de l'activité antithrombine (STA-Stachrom antithrombine III) provient de Diagnostica STAGO. Calculs enzymatiques ont été réalisés avec PRISM 5.0.

### Méthodes

### 1) Test de génération de thrombine (TGA)

Des mesures de génération de thrombine ont été réalisées selon le procédé de Hemker en utilisant 1 pM de Facteur Tissulaire (FT) pour activer la coagulation et en présence de 30 µg / ml CTI pour inhiber l'activation de la phase contact de la coagulation au cours de la période d'incubation (van Veen JJ, Gatt A, Cooper PC, Kitchen S, Bowyer AE, Makris M. Corn trypsin inhibitor in fluorogenic thrombin-generation measurements is only necessary at low tissue factor concentrations and influences the relationship between factor VIII coagulant activity and thrombogram parameters. Blood Coagul Fibrinolysis. 2008 Apr;19(3):183-9). En bref, un mélange de 20 µl de FT, 4 µM phospholipides, et 80 µl de plasma ont été pipettés en triple exemplaires dans une plaque de microtitration. Vingt microlitres de calibrateur Thrombine avec 80 µl de plasma ont également été déposés à la pipette en triple exemplaires dans la plaque. La plaque a ensuite été insérée dans un Varioskan (Thermofisher, Illkirch, France) avec une longueur d'onde d'excitation réglée à 390 nm, avec une longueur d'onde d'émission de 460 nm et une bande passante de 10 nm. Vingt microlitres de FluCaKit (2,5 substrat filuorogène mM (Z-Gly-Gly-Arg-AMC, ZGGR-AMC) avec 0,1 M de CaCl₂) ont été injectés dans tous les puits, démarrant ainsi la réaction. Le signal de fluorescence est lu toutes les 20 secondes pendant 60 min. Les données brutes sur les intensités de fluorescence ont été exportés vers Sigmaplot ® 9.0 pour des calculs mathématiques en utilisant la méthode 3-ondes décrit précédemment (De Smedt E. Advanced thrombinoscopy: PhD thesis, University Maastricht; 2007). Dans la suite du texte :
ETP désigne potentiel de thrombine endogène et correspond à l'aire sous la courbe;
PH représente la hauteur du pic et correspond au niveau maximal de la thrombine;
LT est le temps de latence et correspond au temps pour atteindre 2 nM de thrombine;
PT est le temps au pic et correspond au temps pour obtenir le PH.

Les différents facteurs GDXa, Xa ou Novoseven^{R} sont dilués dans le tampon A comprenant 1% Prionex, 18 mM d'HEPES, 135 mM de chlorure de sodium, pH 7,35 et ajouté à des plasmas hémophiles prétraités au CTI à diverses concentrations.

### 2) Neutralisation de l'anti-thrombine et TFPI par des anticorps spécifiques

Pour la neutralisation de l'antithrombine, un plasma d'hémophilie A sévère a été enrichi avec différentes concentrations d'anticorps IgG de mouton anti antithrombine humaine (1,8, 3, 5, et 7,5 g / l) et mis à incuber pendant une heure à 25°C avant d'être testés en TGA. En parallèle, l'activité antithrombine a été mesurée sur un coagulomètre STAR (Diagnostica Stago) avec des réactifs antithrombine III STA-Stachrom.

Pour la neutralisation du TFPI, le même plasma l'hémophilie A a été mis en contact avec des concentrations différentes d'immunoglobulines de mouton anti-TFPI humain (2,5, 5, 10 et 50 mg/l) avant d'être testés en TGA. Parallèlement, l'activité du TFPI a été déterminée avec le test d'activité Actichrom TFPI selon les instructions du fabricant. En bref, 20 µl de plasma dilué 20 fois ont été incubé à 37°C en présence de 20 µl TF / FVIIa pendant 30 min. Ensuite, le facteur X (FX) a été ajouté et l'ensemble incubé à 37 ° C pendant 15 min avant d'ajouter l'EDTA et Spectrozyme FXa. La réaction a été arrêtée 5 min plus tard en ajoutant l'acide acétique glacial et l'absorbance lue à 405 nm.

### 3) Dosages chromogènes

### 3.1) Détermination des constantes cinétiques des GDXa et Xa

Pour la détermination de l'activité GDXa ou FXa, 0,3 nM d'enzyme est incubée pendant 5 min à 37°C dans un tampon comprenant 1% Prionex, 18 mM d'HEPES, 135 mM de chlorure de sodium, pH 8,4. Puis, le substrat chromogène PNAPEP 1025 est ajouté à des concentrations de 0,33, 0,50, 1, 1,5, et 2,0 mM, et la variation de l'absorbance est enregistrée à 405 nm.

### 3.2) Inhibition enzymatique de l'antithrombine (AT)

Le Xa ou GDXa (1,25 nM) est incubé à 37°C dans le tampon A en présence de concentrations croissantes d'antithrombine (0 à 500 nM). Des aliquotes de 200 microlitres du mélange sont prélevées à différents intervalles de temps, jusqu'à 90 min. Ensuite, 50 µl de substrat chromogène PNAPEP 1025 - 6 mM est ajouté et la variation de l'absorbance est enregistrée.

### 3.3) Inhibition enzymatique du TFPI

L'inhibition de l'activité du GDXa ou FXa par le TFPI a été analysée par l'incubation de l'enzyme de 0,25 nM pour 3h à 25°C dans le tampon A en présence de concentrations croissantes de TFPI (de 0 à 30 nM pour GDXa et de 0 à 10 nM pour FXa) dans un volume final de 200 µl. Ensuite, 50 µl de substrat chromogène PNAPEP 1025 2,5 mM a été ajouté, et la variation de l'absorbance a été enregistré. Le Ki* a été déterminée comme décrit précédemment (Bunce MW, Toso R, Camire RM. Zymogen-like factor Xa variants restore thrombin génération and effectively bypass the intrinsic pathway in vitro. Blood. 2011 Jan 6;117(1):290-8; Baugh RJ, Broze GJ, Jr., Krishnaswamy S. Régulation of extrinsic pathway factor Xa formation by tissue factor pathway inhibitor. J Biol Chem. 1998;273(8):4378-86).

### 3.4) Détermination de la demi-vie plasmatique du GDXa et Xa

La détermination de la demi-vie du GDXa ou du Xa dans le plasma a été réalisée par des ajouts de plasma normal avec 50 nM de GDXa ou de Xa. Le mélange a été ensuite incubé à 37°C. Des aliquotes ont été prélevé de 0 à 60 min et immédiatement dilué 25 fois dans le tampon A avant l'ajout de substrat chromogène PNAPEP 1025 1,5 mM et la détermination de l'activité amidolytique résiduelle, comme décrit précédemment.

### Exemple 2 : Préparation du Facteur Xa modifié

Le plasmide pTT5 est ouvert par digestion à l'aide des enzymes Hindlll - BamHI et les gènes codant pour le peptide signal du TIMP1 avec les sites de restrictions Hindlll et Nhel et du FX dépourvu du domaine Gla avec les sites de restrictions Nhel et BamHI sont insérés, générant le plasmide pTT5-TIMX (pTT5 spTIMP1 gla less FX). La séquence codant pour le FX dépourvu du domaine Gla selon l'invention avec les sites de restrictions Nhel et BamHI a été obtenue par synthèse chimique (GenScript Corporation) Puis un site de reconnaissance pour les furines a été introduit en amont de l'Isoleucine N-terminale de la chaine lourde permettant la sécrétion du GDXa directement dans le milieu de culture pour purification. Le GDXa produit est représenté par l'identifiant de séquence SEQ ID No. 3 pour sa chaine légère et par SEQ ID No. 2 pour sa chaine lourde.

### Exemple 3 : détermination des paramètres cinétiques des GDXa et FXa

Avant d'analyser l'effet de GDXa sur la génération de thrombine, le GDXa et le Fxa ont été caractérisés en utilisant le clivage du substrat chromogène PNAPEP 1025. GDXa a montré une affinité similaire (Km = 0,75 ± 0,05 mM) au FXa (Km = 0,64 ± 0,03 mM) et des propriétés catalytiques similaires : kcat = 290 ± 5 sec-1 pour le GDXa et kcat = 375 ± 8 sec-1 pour le FXa ( Tableau 1). Ces résultats sont cohérents avec les observations antérieures obtenues avec un substrat chromogène S2222 (Skogen WF, Esmon CT, Cox AC. Comparison of coagulation factor Xa and des-(1-44)factor Xa in the assembly of prothrombinase. J Biol Chem. 1984;259(4):2306-10).

**Tableau 1 : propriétés enzymatiques du GDXa et FXa.**

| | | | **Antithrombine** | **TFPI** |
|---|---|---|---|---|
| | Km (mM) | kcat (s-1) | k2 ± SD (10 3.M-1.s-1) | Ki* ± SD (nM) |
| Xa | 0.65 ± | 368 ± | 1.50 ± 0.04 | 0.17 ± 0.031 |
| GDXa | 0.71 ± | 269 ± | 1.57 ± 0.08 | 0.31 ± 0.04 |

Les résultats présentés correspondent à 2 mesures indépendantes réalisées en triplicat en utilisant le substrat chromogène PNAPEP 1025

### Exemple 4 : Influence de la GDXa ou FXa sur la génération de thrombine

A une concentration de 1 pM, le FT est incapable d'induire la production de thrombine dans le plasma d'hémophile A sévère, comme le montre la ligne en pointillés dans la figure 1A. Toutefois, en présence de 50 pM GDXa, la restauration claire de la génération de thrombine a été observée (figure 1 B). GDXa normalise tous les paramètres liés à la génération de thrombine, y compris le potentiel de thrombine endogène (ETP), le temps de latence, la hauteur du pic, et le temps au pic (figure 1B, Tableau 4). La génération de thrombine observée n'était pas un effet direct de GDXa sur le plasma, car aucune thrombine n'a été générée en l'absence de FT (Figure 1B, ligne pointillée). Ceci a été observé dans tous les plasmas d'hémophile testés. En outre, contrairement au GDXa, le FXa a déclenché la génération de thrombine, même en l'absence de FT (figure 1C), car il est directement en mesure de convertir la prothrombine en thrombine.

Pour quantifier l'interférence éventuelle par le clivage direct du substrat ZGGR-AMC par GDXa, des quantités croissantes d'enzyme ont été ajoutés en présence d'une quantité saturante (6 pg / ml) de la lépirudine (inhibiteur de la thrombine). Comme le montre la figure 1D, le signal de fluorescence brute a été totalement aboli en présence de la lépirudine. Dans ces conditions, GDXa a clivé le substrat ZGGR-AMC proportionnellement à sa concentration. A une concentration de 50 nM, le signal final correspondait à 8% de la fluorescence générée en l'absence de lépirudine; à une concentration de 250 nM, le signal représentait à peu près 40% de la fluorescence totale. Cependant, parce que le signal est linéaire, il a été mathématiquement inclus dans le signal du complexe α2-macroglobuline-thrombine dans la méthode 3-ondes utilisée pour calculer les courbes de concentration de thrombine et n'a pas d'incidence sur les résultats de génération de thrombine (De Smedt E. Advanced thrombinoscopy: PhD thesis, University Maastricht; 2007).

La quantité minimale de GDXa capable de restaurer la génération de thrombine dans le plasma de patients atteints d'hémophilie A sévère a été évaluée. Une concentration de 20 nM GDXa a donné dans ce plasma une courbe de génération de thrombine (figure 1 E) similaire à celle observée pour le plasma normal (figure 1A). En outre, 10 nM GDXa ont généré un signal légèrement plus élevé que celui obtenue en présence de 200 nM de rFVIIa (figure 1 F).

### Exemple 5 : Effet des anticorps anti-antithrombine et anti-TFPI sur la génération de thrombine

L'anticorps anti-antithrombine est en mesure d'augmenter massivement l'ETP avec un faible impact sur les paramètres cinétiques (Tableau 2 et figure 2A). A 7,5 g/l d'anticorps anti-antithrombine (activité antithrombine résiduelle 9%), l'ETP a grimpé à 2716 nM.min alors le PH atteint 76 nM. Il contraste avec l'effet sur les paramètres cinétiques (LT = 6,6 min, PT = 31,3 min). De même, comme indiqué précédemment par Erhardtsen et al. (Blocking of tissue factor pathway inhibitor (TFPI) shortens the bleeding time in rabbits with antibody induced haemophilia A. Blood Coagul Fibrinolysis. 1995;6(5):388-94), un anticorps anti-TFPI a aussi été en mesure de restaurer la coagulation dans un plasma d'hémophile. À des concentrations supérieures à 10 mg/l d'anticorps anti-TFPI (activité résiduelle TFPI <30%), tous les paramètres TGA ont été corrigés dans ce plasma de l'hémophilie (Tableau 2). À 10 mg/l (Tableau 2 et figure 2B), ETP et PH ont augmenté respectivement de 209 à 762 nM.min et de 8 à 79 nM. LT et PT ont diminué respectivement de 13,6 à 3,5 min et de 25,9 à 7,2 min.

**Tableau 2 : Influence des anticorps anti-thrombine et anti-TFPI sur la génération de thrombine**

| | **NP** | **HP** | **GDXa 50 nM** | |
|---|---|---|---|---|
| **AT activity (%)** | **-** | **97** | **-** | |
| **TFPI activity (%)** | **-** | **98** | **-** | |
| **ETP (nM.min-1)** | **643** | **209** | **629** | |
| **PH (nM)** | **42** | **7.7** | **43** | |
| **LT (min)** | **4.9** | **13.6** | **3.4** | |
| **PT (min)** | **11.3** | **25.9** | **8.6** | |
| | | | | |

| **anti-AT IgG concentration (g/l)** | **anti-AT IgG 7.5** | **anti-AT IgG 5** | **anti-AT IgG 3** | **anti-AT IgG 1.8** |
|---|---|---|---|---|
| **AT residual activity (%)** | **9** | **24** | **47** | **66** |
| **ETP (nM.min-1)** | **2716** | **1279** | **502** | **360** |
| **PH (nM)** | **76** | **41.4** | **22.5** | **15.5** |
| **LT (min)** | **6.6** | **7.2** | **9.4** | **9.8** |
| **PT (min)** | **31.3** | **24.8** | **21.5** | **23.8** |
| | | | | |

| **anti-TFPI Ig concentration (mg/l)** | **anti-TFPI Ig 50** | **anti-TFPI Ig 10** | **anti-TFPI Ig 5** | **anti-TFPI Ig 2.5** |
|---|---|---|---|---|
| **TFPI residual activity (%)** | **<20** | **29** | **53** | **80** |
| **ETP (nM.min-1)** | **732** | **762** | **593** | **383** |
| **PH (nM)** | **69.5** | **78.8** | **31.9** | **16.5** |
| **LT (min)** | **3.5** | **3.5** | **5** | **7.7** |
| **PT (min)** | **7.5** | **7.2** | **12.7** | **20.3** |

Aux plasmas de patients atteints d'hémophilie A sévère ont été ajoutés des concentrations différentes d'anticorps anti-thrombine humaine ou des anticorps anti-TFPI humains avant le dosage de la génération de thrombine.
HP : Plasma d'hémophile;
PN : plasma normal.
Les activités résiduelles antithrombine et TFPI ont été mesurées selon la méthode décrite dans le matériel et les méthodes.
GDXa : plasma hémophile avec 50 nM GDXa.
Les concentrations d'IgG Anti-AT sont exprimées en g/l.
Les concentrations d'anticorps anti-TFPI sont exprimées en mg/l.

### Exemple 6 : Inhibition enzymatique du GDXa et du FXa par le TFPI et l'antithrombine

Pour l'inhibition enzymatique irréversible par l'antithrombine, les profils d'inhibition du GDXa (1,50 ± 0,04 x 103 M⁻¹.s⁻¹, Figure 2B) et du Xa étaient identiques (k2 = 1,57 ± 0,08 x 103 M⁻¹.s⁻¹, la figure 2A). Le TFPI est un inhibiteur de fixation lente de FXa (26, 27) et, à une faible concentration, un inhibiteur faible de GDXa (28-30). Par conséquent, l'inhibition du FXa et du GDXa ont été comparées (Tableau 1). Le GDXa a montré une plus faible affinité pour TFPI (Ki * = 0,31 ± 0,04 nM, figure 2D) par rapport à FXa (Ki* = 0,17 ± 0,03 nM, figure 2C). En outre, la réalisation de l'équilibre dans cette expérience a été suggéré par l'identité des courbes de titrage après incubation pendant 18 heures.

### Exemple 7 : Demi vie du GDXa et Xa dans le plasma

Considérant l'inhibition du GDXa par le TFPI et l'antithrombine, l'activité résiduelle à la suite de l'ajout dans le plasma de 50 nM de GDXa ou de FXa à 37°C a été évaluée. Comme le montre la figure 4A, l'activité dans le plasma a diminué rapidement, une demi-vie d'environ 1 min 30 sec a été observée, et atteint un plateau après 20 min pour le GDXa ou le FXa comme précédemment indiqué pour FXa (Bunce MW, Toso R, Camire RM. Zymogen-like factor Xa variants restore thrombin génération and effectively bypass the intrinsic pathway in vitro. Blood. 2011 Jan 6;117(1):290-8). Néanmoins, l'effet sur la génération de thrombine a été maintenue au fil du temps, comme à une heure, lorsque l'activité résiduelle de GDXa était à peu près 10% de son activité initiale (figure 4B), la restauration de la génération de thrombine a été maintenue. Après 1 min d'incubation, l'ETP a augmenté de 0 à 610 nM et est resté à 478 nm après 60 min (tableau 3). Une correction similaire a également été observée pour la hauteur du pic maximal (75 nM et 38 nM à 1 et 60 min, respectivement) ainsi que pour le décalage et les temps au pic (tableau 3).

**Tableau 3 : Effet du GDXa après 1 minute et 1 heure d'incubation à 37°C sur un plasma d'hémophile sévère.**

| | **1 min** | **60 min** |
|---|---|---|
| **ETP (nM.min)** | **610** | **478** |
| **PH (nM)** | **75** | **38** |
| **PT (sec)** | **6.6** | **8.8** |
| **LT (sec)** | **1.4** | **2.1** |

50 nM de GDXa a été ajouté à un plasma d'hémophile A sévère et incubé à 37°C durant 1 heure. Des aliquotes ont été prélevées immédiatement et après 1 heure pour mesurer la génération de thrombine.

### Exemple 8 : Influence de la GDXa sur la génération de thrombine chez des patients atteints d'hémophilie A sévère avec et sans inhibiteur et chez des patients atteints d'hémophilie B sévère.

La génération de thrombine a été évaluée dans des échantillons de plasma provenant de cinq différents donneurs atteints d'hémophilie A sévère dont un donneur avec un inhibiteur titré à 50 unités Bethesda, et d'un plasma issu d'un patient atteint d'hémophilie B sévère. La génération de thrombine était quasiment indétectable dans les six plasmas lorsque la coagulation a été déclenchée par 1 pM de FT, alors qu'elle a été restaurée en présence de 20 et 50 nM GDXa. Le tableau 4 montre que les corrections ont été observées à des degrés divers, pour tous les plasmas et pour tous les paramètres liés à la génération de thrombine. En présence de 20 nM GDXa, l'ETP observé était de de 374 ± 128 nM et le PH de 22 ± 11 nM. LT et PT ont diminué respectivement de 5,0 ± 1,5 et 13,9 ± 3,8 min.

En outre, un effet dose a été observé, les valeurs ont augmenté à 533 ± 132 nM pour l'ETP et 46 ± 20 nM pour le PH lorsque 50 nM GDXa ont été ajoutés. LT et PT ont diminué respectivement de 2,8 ± 0,7 et 9,2 ± 2,7 min.

**Tableau 4 : Influence du GDXa sur la génération de thrombine sur 5 différents plasmas issus de patients atteints d'hémophilie A sévère avec ou sans inhibiteur et d'un plasma issu d'un patient atteint d'hémophilie B sévère.**

| **ETP (nM.min)** | **GDXa 0 nM** | **GDXa 20 nM** | **GDXa 50 nM** |
|---|---|---|---|
| **NP** | 611 | - | - |
| **P1 (HA)** | 0 | 536 | 530 |
| **P2 (HA)** | 0 | 268 | 420 |
| **P3 (HA)** | 0 | 207 | 330 |
| **P4 (HA)** | 0 | 466 | 610 |
| **P5 (HA)** | 215 | 331 | 629 |
| **P6 (HA + I)** | 254 | 425 | 539 |
| **P7 (HB)** | 282 | 546 | 668 |
| **Moyenne ± SD** | 125 ± 139 | 374 ± 128 | 533 ± 132 |
| | | | |

| **PH (nM)** | **GDXa** 0 **nM** | **GDXa 20 nM** | **GDXa 50 nM** |
|---|---|---|---|
| **NP** | 43 | ND | ND |
| **P1 (HA)** | 0 | 33 | 40 |
| **P2 (HA)** | 0 | 15 | 26 |
| **P3 (HA)** | 0 | 13 | 25 |
| **P4 (HA)** | 0 | 41 | 78 |
| **P5 (HA)** | 8 | 14 | 43 |
| **P6 (HA + I)** | 15 | 28 | 55 |
| **P7 (HB)** | 13 | 23 | 46 |
| **Moyenne ± SD** | 6 ± 7 | 22 ± 11 | 46 ± 20 |
| | | | |

| **LT (min)** | **HP** | **GDXa 20 nM** | **GDXa 50 nM** |
|---|---|---|---|
| **NP** | 5.7 | ND | ND |
| **P1 (HA)** | 0 | 3.1 | 2.3 |
| **P2 (HA)** | ND | 5.7 | 3.0 |
| **P3 (HA)** | ND | 4.5 | 2.9 |
| **P4 (HA)** | ND | 2.7 | 1.6 |
| **P5 (HA)** | 16 | 7.3 | 3.4 |
| **P6 (HA + I)** | 8 | 4.5 | 2.5 |
| **P7 (HB)** | 17 | 5.5 | 3.3 |
| **Moyenne** ± **SD** | ND | 5.0 ± 1.5 | 2.8 ± 0.7 |
| | | | |

| **PT (min)** | **GDXa 0 nM** | **GDXa 20 nM** | **GDXa 50 nM** |
|---|---|---|---|
| **NP** | 12.3 | ND | ND |
| **P1 (HA)** | 0 | 13.3 | 11.1 |
| **P2 (HA)** | ND | 17.6 | 14.1 |
| **P3 (HA)** | ND | 12.9 | 10.4 |
| **P4 (HA)** | ND | 8.6 | 7.3 |
| **P5 (HA)** | 30.3 | 18.9 | 8.6 |
| **P6 (HA + I)** | 24.4 | 12.4 | 7.4 |
| **P7 (HB)** | 28.9 | 12.8 | 7.3 |
| **Moyenne** ± **SD** | ND | 13.9 ± 3,8 | 9.2 ± 2,7 |

20 ou 20 nM de GDXa ont été ajoutés aux différents plasmas immédiatement testés pour la génération de thrombine.
Px : Plasma x
HA : hémophilie A
HA+I : hémophilie A avec inhibiteur (hémophilie A avec 50 BU d'inhibiteur) HB : hémophilie B

### Exemple 9 : comparatif entre le GDFXa et le facteur VIIa (rVIIa, Novoseven®) selon l'invention sur la génération de thrombine chez des sujets atteints d'hémophilie A.

La Figure 5 montre que le GDXa est beaucoup plus efficace que le rVIIa (Novoseven®) pour corriger la génération de thrombine.

Il faut au moins 500 nM de rVIIa pour corriger selon : ALJAMALI MN, KJALKE M, HEDNER U, EZBAN M, TRANHOLM M. Thrombingeneration and platelet activation induced by rFVIIa (NovoSeven®) and NN1731 in a reconstituted cell-based model mimicking haemophilia conditions. Haemophilia. 2009; 15: 1318-26.

## Revendications

1. Composition pharmaceutique comprenant un facteur Xa modifié (GDXa) - ledit GDXa est un FXa dépourvu de domaine Gla non thrombogène et qui peut se lier à l'inhibiteur de la voie du facteur tissulaire (TFPI)) mais ne possède pas de site de liaison aux phospholipides - pour l'utilisation dans la prévention ou le traitement d'un accident hémorragique chez un patient atteint d'hémophilie A ou B avec ou sans anticorps dirigés contre le facteur VIII ou le facteur IX.

2. Composition pour l'utilisation selon la revendication 1 **caractérisée en ce que** ledit GDXa comprend une délétion du domaine EGF1, une délétion du domaine EGF2 ou une délétion des domaines EGF1 et EGF2.

3. Composition pour l'utilisation selon l'une des revendications 1 ou 2 **caractérisée en ce que** le GDXa présente une chaine lourde intacte.

4. Composition pour l'utilisation selon l'une des revendications 1 à 3 **caractérisée en ce que** le GDXa est représenté par SEQ ID No. 7 ou SEQ ID No. 3, SEQ ID No. 4, SEQ ID No. 5 ou SEQ ID No. 6 pour sa chaine légère et par SEQ ID No. 2 SEQ ID No. 9, SEQ ID No. 10, SEQ ID No.11, SEQ ID No.12, SEQ ID No.13 ou SEQID No.26 pour sa chaine lourde.

5. Composition pour l'utilisation selon l'une des revendications 1 à 3 **caractérisée en ce que** le GDXa est codé par les séquences nucléiques SEQ ID No. 8, SEQ ID No. 16, SEQ ID No. 17, SEQ ID No. 18 ou SEQ ID No. 19 pour sa chaine légère et par SEQ ID No. 15, SEQ ID No. 21, SEQ ID No. 22, SEQ ID No.23, SEQ ID No.24, SEQ ID No.25 ou SEQ ID No.27.pour sa chaine lourde.

6. Composition pour l'utilisation selon l'une des revendications 1 à 5 comprenant 4,5 à 27 µg/kg de GDXa pour une administration par voie systémique, nasale ou parentérale au patient atteint d'hémophilie A ou B avec ou sans anticorps dirigés contre le facteur VIII ou le facteur IX.

## Patentansprüche

1. Pharmazeutische Zusammensetzung, die einen modifizierten Faktor Xa (GDXa) umfasst - wobei der besagte GDXa ein nicht thrombogener FXa ohne Gla-Domäne ist und sich an den Tissue-Factor-Pathway-Inhibitor (TFPI) binden kann, jedoch keine Stelle zur Bindung an Phospholipide besitzt -, zur Verwendung bei der Verhinderung oder der Behandlung eines hämorrhagischen Schlaganfalls bei einem Patienten, der an Hämophilie A oder B erkrankt ist, mit oder ohne Antikörper, die gegen den Faktor VIII oder den Faktor IX gerichtet sind.

2. Zusammensetzung zur Verwendung nach Anspruch 1, **dadurch gekennzeichnet, dass** der besagte GDXa eine Deletion der EGF1-Domäne, eine Deletion der EGF2-Domäne oder eine Deletion der EGF1- und der EGF2-Domäne umfasst.

3. Zusammensetzung zur Verwendung nach einem der Ansprüche 1 oder 2, **dadurch gekennzeichnet, dass** der GDXa eine intakte schwere Kette aufweist.

4. Zusammensetzung zur Verwendung nach einem der Ansprüche 1 bis 3, **dadurch gekennzeichnet, dass** der GDXa durch SEQ ID Nr. 7 oder SEQ ID Nr. 3, SEQ ID Nr. 4, SEQ ID Nr. 5 oder SEQ ID Nr. 6 für seine leichte Kette und durch SEQ ID Nr. 2, SEQ ID Nr. 9, SEQ ID Nr. 10, SEQ ID Nr. 11, SEQ ID Nr. 12, SEQ ID Nr. 13 oder SEQ ID Nr. 26 für seine schwere Kette dargestellt wird.

5. Zusammensetzung zur Verwendung nach einem der Ansprüche 1 bis 3, **dadurch gekennzeichnet, dass** der GDXa von den Nukleinsäuresequenzen SEQ ID Nr. 8, SEQ ID Nr. 16, SEQ ID Nr. 17, SEQ ID Nr. 18 oder SEQ ID Nr. 19 für seine leichte Kette und von SEQ ID Nr. 15, SEQ ID Nr. 21, SEQ ID Nr. 22, SEQ ID Nr. 23, SEQ ID Nr. 24, SEQ ID Nr. 25 oder SEQ ID Nr. 27 für seine schwere Kette codiert wird.

6. Zusammensetzung zur Verwendung nach einem der Ansprüche 1 bis 5, die 4,5 bis 27 µg/kg GDXa umfasst, für eine systemische, nasale oder parenterale Verabreichung an den Patienten, der an Hämophilie A oder B erkrankt ist, mit oder ohne Antikörper, die gegen den Faktor VIII oder den Faktor IX gerichtet sind.

## Claims

1. A pharmaceutical composition comprising a modified Xa factor (GDXa) - said GDXa being an FXa devoid of non-thrombogenic Gla field and which may bind to the Tissue Factor Pathway Inhibitor (TFPI) but does not have a binding site to phospholipids - for the use in preventing and treating a hemorrhagic stroke in a patient suffering from hemophilia A or B with or without antibodies directed against factor VIII or factor IX.

2. The composition for the use according to claim 1, **characterized in that** said GDXa comprises a deletion of the EGF1 domain, a deletion of the EGF2 domain or a deletion of the EGF1 and EGF2 domains.

3. The composition for the use according to any of claims 1 or 2, **characterized in that** the GDXa has an intact heavy chain.

4. The composition for the use according to any of claims 1 to 3, **characterized in that** the GDXa is represented by SEQ ID No. 7 or SEQ ID No. 3, SEQ ID No. 4, SEQ ID No. 5 or SEQ ID No. 6 for its light chain and by SEQ ID No. 2, SEQ ID No. 9, SEQ ID No. 10, SEQ ID No. 11, SEQ ID No. 12, SEQ ID No. 13 or SEQID No. 26 for its heavy chain.

5. The composition for the use according to any of claims 1 to 3, **characterized in that** the GDXa is encoded by the nucleic sequences SEQ ID No. 8, SEQ ID No. 16, SEQ ID No. 17, SEQ ID No. 18 or SEQ ID No. 19 for its light chain and by SEQ ID No. 15, SEQ ID No. 21, SEQ ID No. 22, SEQ ID No. 23, SEQ ID No. 24, SEQ ID No. 25 or SEQ ID No. 27 for its heavy chain.

6. The composition for the use according to any of claims 1 to 5, comprising 4.5 to 27µg/kg of GDXa for a systemic, nasal or parenteral administration to patient suffering from hemophilia A or B with or without antibodies directed against factor VII or factor IX.
